# EUROPÄISCHE PATENTSCHRIFT

(11) **EP 1 605 827 B1**
(45) Veröffentlichungstag und Bekanntmachung des Hinweises auf die Patenterteilung: **28.05.2008**
(21) Anmeldenummer: 04722829.1
(22) Anmeldetag: 24.03.2004
(51) Int. Cl.: A61B 6/14

(54) **ZAHNÄRZTLICHES RÖNTGENSYSTEM MIT ELEKTRONISCHEM SENSOR**
DENTAL X-RAY SYSTEM WITH AN ELECTRONIC SENSOR
SYSTEME DE RADIOGRAPHIE DENTAIRE EQUIPE D'UN DETECTEUR ELECTRONIQUE

(30) Priorität: 24.03.2003 DE 10313043; 27.11.2003 DE 10355431
(43) Veröffentlichungstag der Anmeldung: 21.12.2005
(73) Patentinhaber: Kaltenbach & Voigt GmbH, 88400 Biberach (DE)
(72) Erfinder: HACK, Alexander, 88400 Biberach (DE); ZELLER, Uwe, 88400 Biberach (DE); WEBER, Klaus, 88456 Grodt (DE)
(74) Vertreter: Schmidt-Evers, Jürgen
(86) Internationale Anmeldenummer: PCT/EP2004/003127
(87) Internationale Veröffentlichungsnummer: WO 2004/084731

(56) Entgegenhaltungen:
- WO-A-00/42491
- WO-A-01/22873
- WO-A-01/66012
- WO-A-02/41783
- WO-A-96/03917
- US-A1- 2002 067 407
- US-B1- 6 320 934

## Beschreibung

Die vorliegende Erfindung betrifft ein zahnärztliches Röntgensystem zur digitalen Röntgenuntersuchung.

Röntgenuntersüchungen sind in der Zahnmedizin heutzutage unerlässlich, um eine abschließende Diagnose erstellen zu können. So können beispielsweise Fehlstellungen von Zähnen oder Beschädigungen der Zahnwurzelbereiche oftmals nur anhand von Röntgenbildern erkannt werden. Generell wird dabei zwischen sog. intraoralen Röntgenaufnahmen entschieden, bei denen ein röntgenstrahiungsempfindlicher Sensor im Mundraum eines zu untersuchenden Patienten angebracht ist, sowie Panoramaaufnahmen, bei denen der Patientenkopf vollständig zwischen dem die Strahlungsquelle enthaltenden Rüntgenkopf sowie einem die Röntgenstrahlung erfassenden Sensor angeordnet ist.

Die bei intraoralen Röntgenaufnahmen in dem Mundraum des Patienten angeordneten Sensoren weisen eine Länge und Breite von einigem cm auf. Als Sensoren wurden früher Röntgenfilme verwendet, die von einer Kunststoffhülle umschlossen waren. Nach Belichtung des Sensors musste der Film aus der Tasche entfernt und entwickelt werden bis letztendlich das Ergebnis der Röntgenuntersuchung betrachtet werden konnte.

In jüngster Zeit werden die klassischen Röntgenfilme immer mehr durch elektronische bzw. digitale Röntgensensoren ersetzt. Anstelle eines strahlungsempfindlichen Films wird hierbei ein strahlungsempfindliches Halbleiterelement verwendet, beispielsweise ein CCD- oder CMOS-Chip, das in einzelne Bildbereiche (Pixel) unterteilt ist und die Röntgenstrahlung - genauer gesagt, die mit Hilfe einer dem Halbleiterelement vorgeordneten Leuchtschicht (dem sog. Scintillator) in sichtbares Licht umgewandelte Röntgenstrahlung - erfasst. Die angesprochene Leuchtschicht ist deshalb erforderlich, da das Halbleiterelement für Strahlung aus dem sichtbaren Bereich deutlich empfindlicher ist als für die Röntgenstrahlung selbst. Die von dem Halbleiterchip ausgegebenen Daten können dann von einer Auswerteeinheit ausgelesen werden, die anhand dieser Informationen dann das Röntgenbild erstellt. Der Vorteil dieses digitalen Verfahrens liegt darin, dass die während einer Aufnahme entstehenden Bilddaten unmittelbar zur Verfügung stehen, so dass im wesentlichen zeitgleich das Röntgenbild auf einem Monitor oder Display betrachtet werden kann und nicht zunächst in aufwendiger Weise der Film entwickelt werden muss.

Eine Kamera zum Erstellen digitaler Röntgenaufnahmen ist beispielsweise in der WO 01/66012 A1 beschrieben. Diese bekannte Kamera weist als Besonderheit eine Sensorfläche auf, welche in mehrere Bereiche unterteilt ist, die jeweils für sich einzeln genommen zum Erhalten von Röntgendaten herangezogen werden können. Insbesondere können die Sensorflächen auch parallel ausgelesen werden.

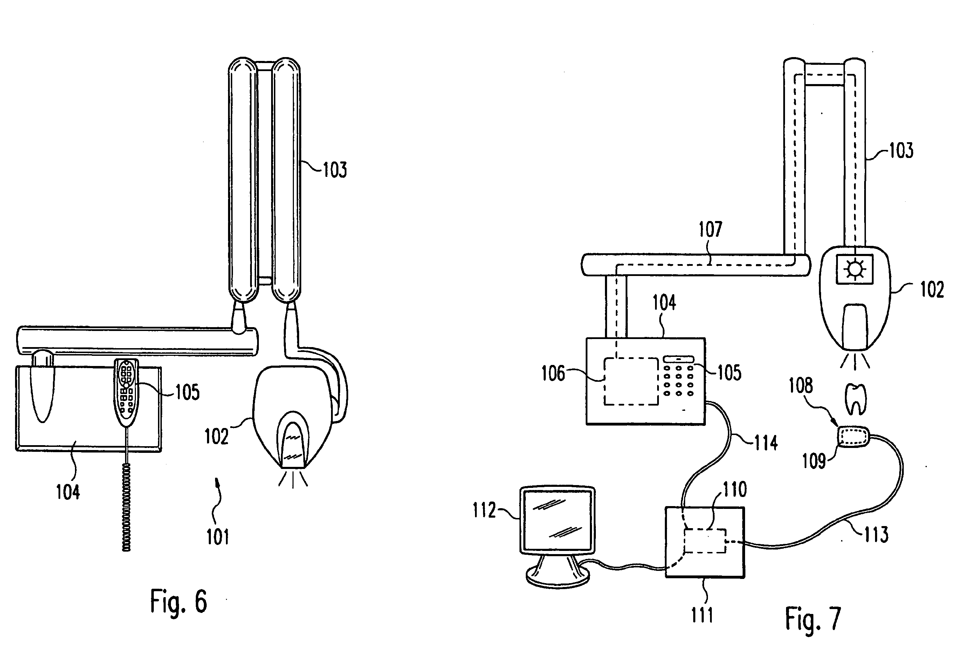

Auch wenn die digitale Röntgentechnologie sehr viele Vorteile bietet, so besteht gegenüber der herkömmlichen klassischen Röntgentechnologie ein Nachteil darin, dass die digitalen Röntgensensoren im Vergleich zu den klassischen Röntgenfilmen mit einer Steuer- und Auswerteelektronik verbunden werden müssen, um die von dem Sensor aufgenommenen Daten auswerten zu können. Die Verbindung zwischen dem Sensor und der Steuer- und Auswerteeinheit erfolgt dabei üblicherweise mit Hilfe eines Kabels, das aus dem Patientenmund herausgeführt ist und zu der Steuer- und Auswerteeinheit verläuft. Darüber hinaus muss die Steuer- und Auswerteelektronik sicherstellen, dass der digitale Sensor zeitgleich mit der Aktivierung der Röntgenstrahlungsquelle ausgelesen wird, dass also das System die komplette Strahlzeit erfasst bzw. erkennt und unmittelbar nach dem Abschalten der Strahlung das Bild zur Verfügung stellt. Die in diesem Zusammenhang auftretenden Schwierigkeiten sollen zunächst anhand der beiden Figuren 6 und 7 erläutert werden, welche ein digitales zahnärztlichen Röntgensystem nach dem Stand der Technik darstellen. Fig. 6 zeigt dabei ausschließlich das Röntgengerät, während hingegen Fig. 7 ein komplettes Röntgensystem incl. der Steuer- und Auswerteelektronik für den digitalen Sensor zeigt.

Das in Fig. 6 allgemein mit dem Bezugszeichen 101 bezeichnete Röntgengerät weist als wesentlichen Bestandteil einen die Strahlungsquelle enthaltenden Röntgenkopf 102 auf, der über ein Gestänge 103 einer Halterung bewegbar mit einer - beispielsweise an einer Wand oder Decke montierten - Zentraleinheit 104 verbunden ist. Die Zentraleinheit 104 beinhaltet - wie Fig. 7 entnommen werden kann - eine Steuerungselektronik 106, welche für die Ansteuerung des Röntgenkopfes 102, insbesondere der darin angeordneten Röntgenstrahlungsquelle verantwortlich ist. Die hierzu erforderlichen Informationen werden über Leitungskabel 107 übertragen, welche innerhalb des Gestänges 103 verlaufen. Die Kabel 107 weisen darüber hinaus auch die Stromversorgungsleitungen für die Röntgenstrahlungsquelle auf.

Die Zentraleinheit 104 weist Eingabeelemente 105 auf, über die ein Arzt oder eine sonstige Person, welche das Röntgengerät bedient, Parameter für die durchzuführende Röntgenuntersuchung (beispielsweise Strahlzeit, Strahldauer, Röhrenstrom (mA) und Röhrenspannung (kV)) eingeben kann. Anhand dieser Informationen übermittelt die Steuerungselektronik 106 dann die entsprechenden Signale an den Röntgenkopf 102.

Die bei einer Röntgenuntersuchung von dem Röntgenkopf 102 ausgehenden Strahlen werden von einem hinter dem zu untersuchenden Objekt - in Fig. 7 schematisch durch den Zahn dargestellt - angeordneten Sensor 108 erfasst, der als wesentliches Bauteil einen strahlungsempfindlichen Halbleiter-Chip 109 aufweist, der in einem flachen Gehäuse angeordnet ist. Die von dem CCD- oder CMOS-Chip 109 erfassten Informationen werden über ein Kabel 113 an eine Steuer- und Auswerteelektronik 110 weitergeleitet, welche in einem separaten Gehäuse 111 angeordnet ist. Die Steuer- und Auswerteelektronik 110 ist zum einen für die Ansteuerung des Sensors 109 erforderlich, zum anderen wertet sie die Bildinformationen aus und übermittelt diese an ein PC-System 112, auf dem dann das digitale Röntgenbild betrachtet und archiviert werden kann.

Ein Nachteil bei diesem klassischen Aufbau eines digitalen Röntgenssystems besteht darin, dass üblicherweise das Gehäuse 111 für die Steuer- und Auswerteelektronik 110 des Sensors 108 unabhängig von der Zentraleinheit 104 des Röntgengeräts angeordnet ist. Dies hat zur Folge, dass das Anschlusskabel 113 für den Sensor 108 verhältnismäßig lang ist und zumindest teilweise durch den Untersuchungsraum geführt ist. Hierbei besteht zum einen die Gefahr, dass das Kabel den Boden berühren kann, was aus hygienischen Gründen nicht erwünscht ist, zum anderen kann es ein Hindernis darstellen, über welches eine Person stolpern könnte, wobei der Sensor 108 oder die an dem Sensoranschluss befindliche Elektronik beschädigt werden könnte.

Ein weiterer Nachteil der Trennung von Zentraleinheit 104 des Röntgengeräts 101 und Steuer- und Auswerteelektronik 110 für den Sensor 108 besteht darin, dass ein zusätzliches Kabel 114 erforderlich ist, welches von der Zentraleinheit 104 zu dem Gehäuse 111 für die Steuer- und Auswerteelektronik 110 verläuft und sicherstellt, dass der Sensor 108 synchron mit der Aktivierung der Röntgenstrahhmgsquelle ausgelesen wird. Auch dieses Kabel 114 stellt ein Hindernis dar. Zwar werden Ansätze verfolgt, den. Sensor 109 durch die Sensorstrahlung selbst zu triggern, diese Verfahren sind allerdings verhältnismäßig aufwendig und gewährleisten im Vergleich zu der Übermittlung eines. Aktivierungssignals über ein Kabel keine absolute Sicherheit.

Um die soeben geschilderten Probleme zu umgehen, ist es aus der WO 96103917 A1 bereits bekannt, einen Teil der Steuer- und Auswerteelektronik für den digitalen Sensor in die Zentraleinheit des Röntgengerätes zu integrieren und die Leitung zwischen dem Sensor und der Elektronik zu einem Großteil innerhalb des Gestänges verlaufen zu lassen. Am Röntgenkopf selbst befindet sich dann eine Steckbuchse, in die ein entsprechendes externes Anschlusskabel, welches zu dem digitalen Sensor führt, angeschlossen werden kann. Der Vorteil bei dieser Variante besteht darin, dass das externe Anschlusskabel für den Sensor deutlich kürzer ist als bei dem in Fig. 7 dargestellten System, so dass dieses Kabel kein Hindernis mehr für Personen innerhalb des Untersuchungsraumes darstellt.

Ein Wechsel zwischen verschiedenen Sensoren wird auch bei einem aus der WO 02/41783 A1 bekannten Röntgensystem ermöglicht, bei dem digitale Röntgensensoren mit Hilfe eines Steckers unmittelbar an einen PC angeschlossen werden können. Der Stecker weist hierzu eine interne Elektronik auf, über welche ein Empfang und eine Weiterverarbeitung der von dem Sensor erhaltenen Signale erfolgt.

Schließlich beschreibt die US 2002/0067407 A1 ein zahnärztliches Röntgensystem, bei dem digitale Sensoren an eine Zentraleinrichtung angeschlossen werden. Hierzu ist ein spezielles Interface vorgesehen, welches in einen entsprechenden Port des Zentralgeräts eingeschoben wird. Die genauere Funktion dieses Interface wird allerdings nicht beschrieben.

Ausgehend von dem bekannten Stand der Technik liegt der vorliegenden Erfindung die Aufgabe zugrunde, ein zahnärztliches Röntgensystem anzugeben, welches dem Zahnarzt ermöglicht, flexibel digitale Sensoren unterschiedlicher Größe oder Ausgestaltung einzusetzen.

Die Aufgabe wird durch ein Röntgensystem, welches die Merkmale des unabhängigen Anspruch 1 aufweist, gelöst.

Gemäß der vorliegenden Erfindung besteht das zahnärztliche Röntgensystem zunächst aus einem Röntgengerät mit einer in einem Röntgenkopf angeordneten Strahlungsquelle zum Erzeugen einer Röntgenstrahlung sowie aus einem elektronischen Sensor zum Erfassen der Röntgenstrahlung, wobei der Sensor über einen Steckanschluß lösbar mit einer Zentraleinheit des Röntgensystems verbunden werden kann. Dabei ist erfindungsgemäß die Ansteuerelektronik für den digitalen Sensor in den Stecker integriert.

Die vorliegende Erfindung geht somit im Vergleich zu dem bekannten Stand der Technik insofern einen Schritt weiter, als die für den Betrieb des Sensors erforderliche Ansteuerelektronik in den Steckeranschluss des Kabels verlegt wird, das zu dem digitalen Sensor fuhrt. Dies ist möglich, da mittlerweile die entsprechende Ansteuerelektronik für den Halbleiter-Chip des Sensors, z.B. die sog. CCU (CCD control unit) oder die Elektronik zur Ansteuerung eines CMOS-Chips derart miniaturisiert werden kann, dass sie nur noch sehr wenig Platz in Anspruch nimmt. So besteht die Möglichkeit, diese Elektronikbauteile derart zu gestalten, dass sie die Größe einer halben Streichholzschachtel in Anspruch nehmen und lediglich wenige mm hoch sind. Der versorgungsseitige Anschluss für den Stecker könnte dann beispielsweise wiederum in den Röntgenkopf oder in unmittelbarer Nähe davon in das Gestänge des Röntgengeräts integriert werden.

Der Vorteil der Zuordnung der Ansteuerelektronik in das Anschlusskabel für den digitalen Sensor besteht darin, dass der Sensor mit der zugehörigen Elektronik als einfache Baueinheit entfernt und durch eine neue Kombination ausgetauscht werden kann, so dass grundsätzlich für jeden verwendeten digitalen Sensor die geeignete Steuerelektronik vorliegt. Für den Zahnarzt ist es hierdurch möglich, ohne größeren Aufwand die Sensoren zu wechseln und beispielsweise einen neuen Sensor mit größeren Abmessungen zu verwenden.

Weiterhin wird ein einfacher Wechsel zwischen zwei Sensoren unterschiedlicher Größe dadurch ermöglicht, dass das Röntgensystem mit mehreren verschiedenen Sensoren ausgestattet ist, die während einer Röntgenaufnahme gleichzeitig ausgelesen werden, wobei die Steuer- und Auswerteelektronik der Sensoren automatisch erkennt, welcher Sensor im Strahlungsbereich der Röntgenstrahlung angeordnet ist. Alternativ dazu besteht auch die Möglichkeit, die erfaßten Bilddaten beider Sensoren an einen PC zu übertragen und mit Hilfe einer entsprechenden Software eine Entscheidung durchzuführen, welche Bilddaten relevant sind.

Der Benutzer des Röntgensystems kann somit frei zwischen den zur Verfügung stehenden Sensoren wählen und muss nicht zunächst dem Gerät Informationen hinsichtlich des Typs des verwendeten Sensors mitteilen. Stattdessen erkennt das System anhand der übermittelten Daten automatisch, welcher Sensor gerade verwendet wird, wodurch eine besonders einfache Bedienung ermöglicht wird.

Weiterbildungen der Erfindung sind Gegenstand der Unteransprüche. Diese betreffen in erster Linie die Integration der Steuer- und Auswerteelektronik in das Röntgengerät. Vorzugsweise werden die Röntgenstrahlungsquelle sowie der Sensor und dessen Ansteuerelektronik von einer gemeinsamen Stromquelle versorgt. Darüber hinaus ist vorzugsweise eine Verbindungsleitung vorgesehen, über welche der Ansteuerelektronik für den digitalen Sensor mitgeteilt wird, zu welchem Zeitpunkt die Röntgenstrahlungsquelle aktiviert wird, so dass synchron dazu automatisch der Sensor ausgelesen werden kann.

Eine andere Weiterbildung betrifft die Lagerung des bzw. der Sensoren..So ist vorzugsweise unmittelbar an dem Röntgenkopf eine Ablage für den Sensor vorgesehen, die gemäß einem besonders bevorzugten Ausführungsbeispiel abnehmbar ausgestaltet und sterilisierbar ist. In diesem Zusammenhang ist zu berücksichtigen, dass der Sensor üblicherweise vor der Benutzung mit einer Einweg-Umhüllung und/oder einem Haltesystem versehen wird und dann mehrere Aufnahmen nacheinander erstellt werden. Muß der Ablauf unterbrochen werden, so muß die Möglichkeit bestehen den Sensor kurzzeitig abzulegen. Hierbei wird allerdings die Ablage kontaminiert, so dass sie anschließend gereinigt und sterilisiert werden muß, was durch die abnehmbare Ausgestaltung erleichtert wird.

Nachfolgend soll die Erfindung anhand der beiliegenden Zeichnungen näher erläutert werden. Es zeigen:
- Fig. 1: ein erstes Ausführungsbeispiel eines zahnärztlichen digitalen Röntgensystems gemäß der vorliegenden Erfindung;
- Fig. 2: das in Fig. 1 dargestellte Röntgensystem in schematischer Darstellung;
- Fig. 3: eine vergrößerte Darstellung eines digitalen Sensors sowie des zugehörigen Anschlußkabels;
- Fig. 4: einen Röntgenkopf gemäß einem zweiten Ausführungsbeispiel, welches nicht Teil des Erfindung ist;
- Fig. 5: eine alternative Ausführungsform des Röntgensystems; und
- Fig. 6 und 7: ein Röntgengerät bzw. ein Röntgensystem gemäß dem Stand der Technik**.**

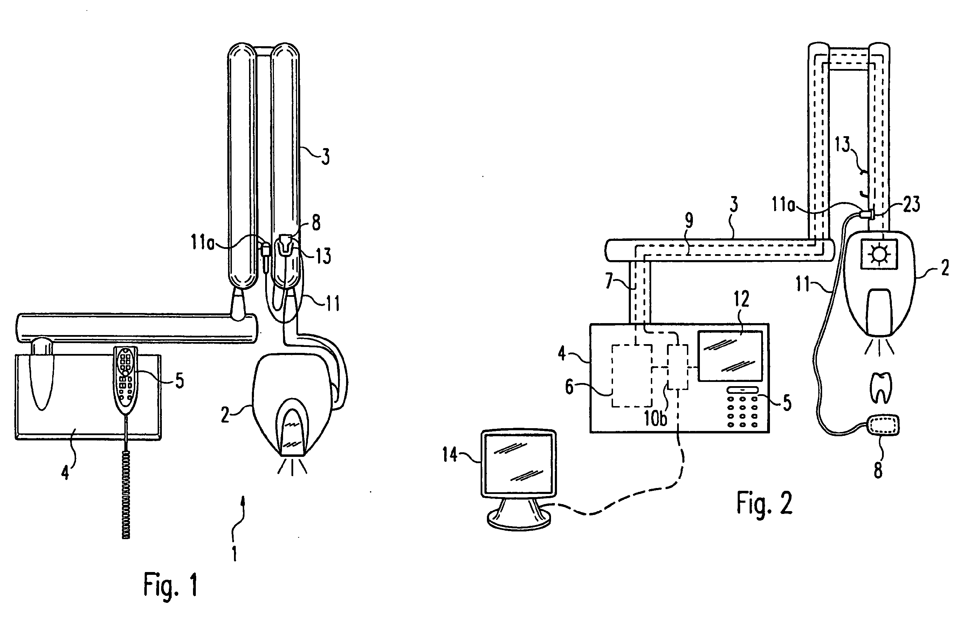

Das in den Fig. 1 und 2 dargestellte Röntgensystem 1 weist wiederum zunächst einen die Röntgenstrahlungsquelle enthaltenden Röntgenkopf 2 auf, der über ein Gestänge 3 einer Halterung mit einer Zentraleinheit 4 verbunden ist. Die Zentraleinheit 4 ist im dargestellten Ausführungsbeispiel an einer Wand befestigt, sie könnte allerdings ebenso an einer Decke des Untersuchungsraumes befestigt oder als eine separate Konsole ausgeführt sein. An der Außenseite der Zentraleinheit 4 sind Eingabeelemente 5 zur Eingabe gewünschter Untersuchungsparameter (Strahlzeit, Strahldauer, Röhrenstrom (mA), Röhrenspannung (kV) etc.) angeordnet. Im Inneren weist sie zunächst eine Steuereinheit 6 auf, welche für die Ansteuerung der Röntgenstrahlungsquelle in dem Röntgenkopf 2 verantwortlich ist und zu diesem Zweck über eine durch das Gestänge 3 verlaufende Leitung 7 mit dieser verbunden ist.

Wesentliches Merkmal des erfindungsgemäßen Röntgensystems ist, dass der Röntgensensor 8 nun nicht mehr mit einem separaten Gehäuse verbunden ist, sondern stattdessen über ein verhältnismäßig kurzes Kabel 11 an eine Steckbuchse 23 angeschlossen ist, die in dem Gestänge 3 in unmittelbarer Nähe des Röntgenkopfes 2 angeordnet ist. Aus dieser Anordnung ergibt sich der Vorteil, dass das Anschlusskabel 11 für den Sensor 8 nur verhältnismäßig kurz ist und nicht mehr als Hindernis durch den Untersuchungsraum verläuft. Die kurze Länge des Kabels 11 hat darüber hinaus den Vorteil, dass der Sensor 8 nicht mehr auf den Boden fallen kann. Im dargestellten Beispiel ist an dem Gestänge 3 ferner eine Ablage 13 vorgesehen, in welche der Sensor 8 bei Nichtgebrauch abgelegt werden kann.

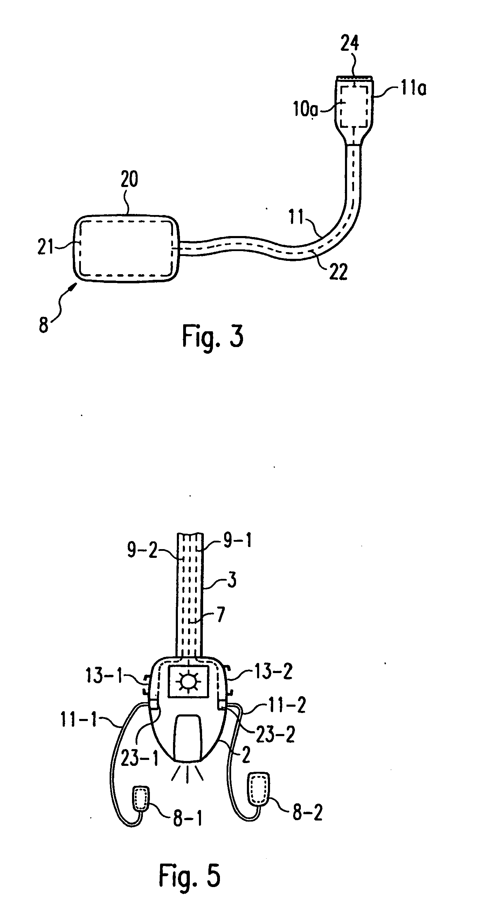

Zur Ansteuerung des digitalen Sensors 8 ist wiederum eine entsprechend Elektronik erforderlich. Gemäß der vorliegenden Erfindung ist allerdings nunmehr diese Ansteuerelektronik 10a in das Kabel 11, genauer in den Anschlussstecker 11a des Kabels 11 integriert, wie in Fig. 3 näher dargestellt ist.

Der Sensor 8, der im wesentlichen aus einem röntgenstrahlungs-empfindlichen Halbleiterelement 21 (einem CCD- oder einem CMOS-Chip) besteht, das in einem flachen, rechteckförmigen Gehäuse 20 angeordnet ist, wird dabei über eine Ansteuerelektronik 10a angesteuert, welche innerhalb des Steckers 11a am sensorfernen Ende des Kabels 11 angeordnet ist. Die Ansteuerelektronik 10a, welche die Ansteuerung des Halbleiterelements 21 sowie das Auslesen der Bildinformationen regelt, ist über eine Vielzahl von Leitungen 22, die innerhalb des Kabels 11 verlaufen, mit dem Halbleiterelement 21 verbunden. Aufgrund der nunmehr bestehenden Möglichkeit zur Miniaturisierung derartiger Elektronikelemente weist die Ansteuerelektronik 10a derart geringe Abmessungen auf, dass sie ohne großen Aufwand in den Stecker 11a integriert werden kann. Die der Ansteuerelektronik 10a extern, d.h. von der Zentraleinheit 4 zugeführten Trigger-Signale zur Aktivierung des Sensors 8 oder die von dem Sensor 8 empfangenen und zu übertragenden Bildinformationen werden dann über entsprechende Anschlusskontakte 24 des Steckers 11a weitergeleitet.

Wesentlich ist, dass durch die Integration der Ansteuerelektronik 10a in den Stecker 11a weitere Elektronik-Komponenten, welche an der Ansteuerung des Sensors 8 beteiligt sind, beispielsweise die in Fig. 7 mit dem Bezugszeichen 110 versehene Elektronik, entfallen können. Der Sensor 8 könnte somit beispielsweise auch unmittelbar an einen PC oder ein PC-Peripheriegerät (z.B. einen USB-Hub) angeschlossen werden. Hierdurch unterscheidet sich der Gegenstand der vorliegenden Erfindung von bereits bekannten Systemen, bei denen zwar bereits ein Teil der Elektronik des Sensors in den Stecker integriert ist, die allerdings auf weitere externe Elektroniken für den Betrieb des Sensors angewiesen sind.

Vorzugsweise wird für den Stecker 11a ein standardisiertes System verwendet, welches eine hohe Zahl von Steckzyklen ermöglicht. Hierfür bieten sich beispielsweise USB-Anschlüsse oder metallische Rundverbinder an. Es können allerdings auch Anschlüsse gemäß anderen PC-Standards verwendet werden, insbesondere auch sog. Firewire-Anschlüsse. Im Übrigen könnte der Stecker 11a auch die Leitungen für eine ggf. erforderliche Stromversorgung des Sensors 8 beinhalten, wobei vorzugsweise die Röntgenstrahlungsquelle und der Sensor 8 von der gleichen Stromversorgungsquelle versorgt werden.

Gemäß einem besonders bevorzugten Ausführungsbeispiel ist der Stecker 11a bzw. 23 als USB-Anschluß ausgebildet und die darin enthaltene Ansteuerelektronik 10a für den Sensor 8 stellt ein USB-Gerät dar. Hierbei ist allerdings zu berücksichtigen, dass für eine USB-Teilstrecke eine maximale Länge von 5m zugelassen und andernfalls die Verwendung eines Verstärkers - eines sog. USB-Hubs - erforderlich ist. Da die Armlänge des Gestänges 3 und damit die Länge des Verbindungskabels zwischen dem Stecker 11a und der Zentraleinheit in etwa 3m beträgt, ist vorzugsweise zur Gewährleistung eines sicheren Datenverkehrs vorgesehen, einen entsprechenden USB-Hub zu verwenden, der beispielsweise Bestandteil der später noch beschriebenen Einheit 10b sein kann. In diesem Zusammenhang ist anzumerken, dass aus US 6,134,298 bereits bekannt ist, einen intraoralen digitalen Sensor mittels USB-Anschluß an einen Rechner anzuschließen. Allerdings ist bei diesem bekannten System nicht vorgesehen, die Ansteuerelektronik für den Sensor in den Stecker zu verlagern.

Auch wenn die Ansteuerung des Sensors 8 über die in den Stecker 11a integrierte Ansteuerelektronik 10a erfolgt, so müssen dieser Elektronik 10a dennoch externe Trigger-Signale übermittelt werden, welche ein Auslesen des Halbleiterchips 21 des Sensors 8 initiieren. Hierfür ist innerhalb der Zentraleinheit 4 des Röntgengerätes eine Signal-Einheit 10b vorgesehen, welche mit der Steuereinheit 6 für den Röntgenkopf 2 bzw. für die Röntgenstrahlungsquelle in Verbindung steht und bei einer Aktivierung der Röntgenstrahlungsquelle automatisch ein Startsignal an die Ansteuerelektronik 10a für den Sensor 8 übermittelt, welche ein Auslesen der Bildinformationen initiiert. Die Einheit 10b ist hierzu über eine ebenfalls durch das Gestänge 3 verlaufende Leitung 9 mit der Ansteuerelektronik 10a verbunden. Die Verbindung zwischen der Einheit 10b und der Steuereinheit 6 für das Röntgengerät bzw. die Röntgenstrahlungsquelle hat darüber hinaus zum Vorteil, dass ein bidirektionaler Datenaustausch ermöglicht wird, der eine optimale Abstimmung zwischen Sensorsystem und Röntgenstrahlungsgenerator hinsichtlich der Dauer und Stärke der Belichtung während einer Untersuchung ermöglicht.

Wichtig ist in diesem Zusammenhang, dass die Signal-Einheit 10b ausschließlich mit der Ansteuerelektronik 10a kommuniziert um ein zur Röntgenstrahlen synchrones Auslesen des Sensors 8 zu gewährleisten. An der Ansteuerung des Sensors 8 selbst ist die Signal-Einheit 10b jedoch nicht beteiligt.

Darüber hinaus kann die Einheit 10b auch für das Auswerten der Bildinformationen, die von dem Sensor 8 bzw. dessen Ansteuerelektronik 10a übermittelt wurden, verantwortlich sein. Diese Bildinformationen können dann beispielsweise unmittelbar an ein Display 12 weitergeleitet werden, welches bei dem in Fig. 2 dargestellten Ausführungsbeispiel ebenfalls in die Zentraleinheit 4 des Röntgensystems integriert wurde. Alternativ hierzu können die von dem Sensor 8 bzw. dessen Ansteuerelektronik 10a übermittelten Bildinformationen allerdings von der Einheit 10b auch ohne weitere Bearbeitung an ein externes PC-System 14 bzw. ein zentrales Datenverarbeitungssystem der Praxis oder des Krankenhauses weitergeleitet und dort verarbeitet, betrachtet und archiviert werden. Hierbei kann auch der in der Einheit 10b ggf. vorgesehene USB-Hub zum Einsatz kommen. Die Verbindung zwischen dem Röntgensystem 1 bzw. der Zentraleinheit 4 und dem externen PC-System kann dabei - wie dargestellt - über ein Kabel, das ggf. Bestandteil eines krankenhausinternen Netzwerkes ist, erfolgen, es bestünde allerdings auch die Möglichkeit, die Daten drahtlos, z.B. entsprechend dem Bluetooth-Standard zu übertragen. Ein andere Möglichkeit der Übermittlung der Daten besteht auch darin, diese an der Zentraleinheit 4 auf einem Speichermedium zwischenzuspeichern und das Speichermedium dann auf dem PC-System auszulesen, wobei sich bei dieser Lösung insbesondere die Verwendung von sog. Memory-Sticks oder anderen bekannten Speicherkarten anbietet.

Der Vorteil der Integration der Ansteuerelektronik 10a in den Stecker 11a des Anschlusskabels 11 für den Sensor 8 besteht darin, dass der Sensor 8 und die zugehörige Elektronik 10a gewissermaßen eine Einheit bilden, die optimal aufeinander abgestimmt ist. Soll ein anderer Sensor verwendet werden, so erfolgt dies durch ein Austauschen des Anschlusskabels, wodurch gleichzeitig auch eine auf den neuen Sensor abgestimmte Ansteuerelektronik angeschlossen wird.

Auf diese Weise kann einfach und schnell ein Wechsel zwischen Sensoren unterschiedlicher Art und Größe erfolgen. Die Einheit 10b in dem Zentralgerät 4 des Röntgensystems wiederum ermöglicht, dass ohne großen Aufwand ein synchrones Auslösen des Sensors 8 bei einer Aktivierung der Röntgenstrahlungsquelle erfolgen kann. Ein zusätzliches externes Verbindungskabel zwischen der Steuereinheit für den Röntgengenerator und der Ansteuerelektronik für den Sensor ist ebenfalls nicht mehr erforderlich.

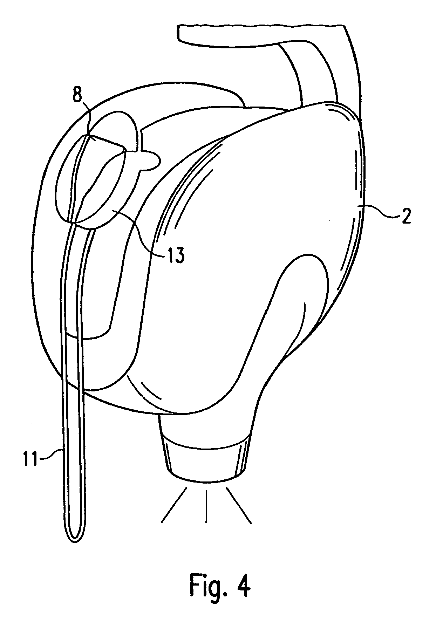

Fig. 4 zeigt ein Ausführungsbeispiel, welches nicht der Teil der Erfindung ist für einen Röntgenkopf 2, welches sich dadurch auszeichnet, dass der Sensor 8 mit seinem Kabel 11 unmittelbar an den Röntgenkopf 2 angeschlossen werden kann. Hierzu ist in dem Gehäuse des Röntgenkopfs 2 eine Steckbuchse vorgesehen, welche unterhalb der Ablage 13 für den Sensor 8 angeordnet und dementsprechend in der Darstellung nicht zu sehen ist. Hinsichtlich der technischen Ausführungen des Anschlusses für den Sensor 8 gleicht das dargestellte Ausführungsbeispiel dem Ausführungsbeispiel der Fig. 1 und 2, d.h., der Stecker ist wiederum bevorzugt als USB-Gerät ausgeführt, kann allerdings auch ein Firewire-Anschluß oder ein Anschluß gemäß einem anderen PC-Standard sein.

Eine weitere Besonderheit dieses zweiten Ausführungsbeispiels besteht darin, dass die Ablage 13 für den Sensor 8 abnehmbar ausgestaltet und sterilisierbar ist. Hierbei ist zu berücksichtigen, dass der Sensor 8 üblicherweise vor der Benutzung mit einer Einweg-Umhüllung und/oder einem Haltesystem versehen wird und dann mehrere Aufnahmen nacheinander erstellt werden. Muß der Ablauf unterbrochen werden, so muß die Möglichkeit bestehen den Sensor 8 kurzzeitig abzulegen. Hierbei wird allerdings die Ablage 13 kontaminiert, so dass sie anschließend gereinigt und sterilisiert werden muß. Die abnehmbare Ausgestaltung der Ablage 13 und die Verwendung eines sterilisierbaren Materials trägt diesem Umstand Rechnung. Selbstverständlich könnte diese abnehmbare und sterilisierbare Ausgestaltung der Ablage 13 auch bei den anderen Ausführungsbeispielen der vorliegenden Erfindung vorgesehen sein.

Fig. 5 zeigt noch eine alternative Ausführungsform, welche ebenfalls einen leichten und schnell durchzuführenden Wechsel zwischen zwei Sensoren ermöglicht.

In dem dargestellten Beispiel weist der Röntgenkopf 2 zwei Anschlüsse 23-1 und 23-2 für jeweils zwei Anschlusskabel 11-1, 11-2 mit entsprechenden Sensoren 8-1, 8-2 auf. Die Sensoren 8-1, 8-2, die sich im dargestellten Ausführungsbeispiel hinsichtlich ihrer Größe unterscheiden, sind jeweils über eine eigene in dem Gestänge 3 verlaufende Leitung 9-1 bzw. 9-2 mit der in der Zentraleinheit 4 angeordneten Einheit 10b verbunden und in der Lage, parallel Daten auszulesen und zu übermitteln. Vorzugsweise ist wie bei den in den Fig. 1 bis 4 dargestellten Ausführungsbeispielen wiederum die Ansteuerelektronik für die Sensoren 8-1 bzw. 8-2 in die Anschlusskabel 11-1, 11-2 bzw. Stecker integriert.

Die Besonderheit besteht bei dem Ausführungsbeispiel gemäß Fig. 5 darin, dass die zur Auswertung der Signale beider Sensoren 8-1 und 8-2 vorgesehene Einheit 10b anhand der von den Sensoren 8-1, 8-2 bzw. deren Ansteuerelektroniken übermittelten Daten automatisch erkennt, welcher der beiden Sensoren 8-1 bzw. 8-2 einer Röntgenstrahlung ausgesetzt wurde. Soll nun eine Röntgenaufnahme erfolgen, so kann ein Benutzer des Röntgensystems frei einen der beiden Sensoren 8-1 bzw. 8-2 wählen, ohne zuvor manuell eingeben zu müssen, welcher Röntgensensor gerade verwendet werden soll. Statt dessen kann er den gewählten Sensor unmittelbar in den Mundraum des Patienten einführen und die Röntgenaufnahme durchführen, da von der Auswerteeinheit automatisch die Bildinformationen des belichteten Sensors erfasst und auf dem Display dargestellt werden.

Alternativ dazu besteht auch die Möglichkeit, beide Sensorsignale an ein externes PC-System weiterzuleiten und erst dort - ggf. durch die Unterstützung einer geeigneten Software - festzulegen, welche Sensorsignale letztendlich verwendet werden sollen. Ferner ist anzumerken, dass nicht zwangsläufig beide Sensorkabel 11-1 und 11-2 an die an dem Röntgenkopf 2 bzw. dem Gestänge 3 angeordnete(n) Steckbuchse(n) 23-1, 23-2 angeschlossen werden müssen. So bestünde auch die Möglichkeit, einen der beiden Sensoren 8-1, 8-2, der z.B. weniger häufig verwendet wird, unmittelbar an das externe PC-System anzuschließen.

Die dargestellten Ausführungsbeispiele eröffnen somit die Möglichkeit, auf einfache Weise zwischen mehreren beliebigen Sensoren zu wählen bzw. einen Sensor durch einen anderen zu ersetzen. Damit wird eine besonders, einfache Handhabung des gesamten Röntgensystems ermöglicht.

## Patentansprüche

1. Zahnärztliches Röntgensystem mit einer in einem Röntgenkopf (2) angeordneten Strahlungsquelle zum Erzeugen einer Röntgenstrahlung sowie einem elektronischen Sensor (8) zum Erfassen der Röntgenstrahlung, wobei der Sensor (8) über einen Steckanschluss (11a, 23) lösbar mit einer Zentraleinheit (4) des Röntgensystems verbindbar ist,
**dadurch gekennzeichnet,**
**dass** die für den Betrieb des Sensors (8) erforderliche Ansteuerelektronik (10a) in einen dem Sensor (8) zugeordneten und mit diesem über ein externes Anschlusskabel (11) verbundenen Stecker (11a) integriert ist.

2. Zahnärztliches Röntgensystem nach Anspruch 1,
**dadurch gekennzeichnet,**
**dass** eine Anschlussbuchse (23) für den Stecker (11a) in dem Röntgenkopf (2) oder in einem den Röntgenkopf (2) tragenden Gestänge (3) einer Halterung in unmittelbarer Nähe des Röntgenkopfes (2) angeordnet ist.

3. Zahnärztliches Röntgensystem nach Anspruch 2,
**dadurch gekennzeichnet,**
**dass** eine oder mehrere Verbindungsleitungen (9) von der Anschlussbuchse (23) zu der Zentraleinheit (4) des Röntgensystems innerhalb (3) des Gestänges verlaufen.

4. Zahnärztliches Röntgensystem nach Anspruch 2 oder 3,
**dadurch gekennzeichnet,**
**dass** an dem Röntgenkopf (2) oder dem Gestänge (3) eine Ablage (13) für den Sensor (8) vorgesehen ist.

5. Zahnärztliches Röntgensystem nach Anspruch 4,
**dadurch gekennzeichnet,**
**dass** die Ablage (13) abnehmbar ist.

6. Zahnärztliches Röntgensystem nach Anspruch 4 oder 5.
**dadurch gekennzeichnet,**
**dass** die Ablage (13) aus einem sterilisierbaren Material besteht.

7. Zahnärztliches Röntgensystem nach einem der Ansprüche 1 bis 6,
**dadurch gekennzeichnet,**
**dass** der Steckanschluss (11a, 23) einen USB-Anschluss beinhaltet.

8. Zahnärztliches Röntgensystem nach Anspruch 7,
**dadurch gekennzeichnet,**
**dass** die in den Stecker (23) integrierte Ansteuerelektronik (10a) als USB-Gerät ausgestaltet ist.

9. Zahnärztliches Röntgensystem nach einem der Ansprüche 1 bis 6,
**dadurch gekennzeichnet,**
**dass** der Steckanschluss (11a, 23) einen Firewire-Anschluss oder einen Anschluss gemäß einem anderen PC-Standard beinhaltet.

10. Zahnärztliches Röntgensystem nach einem der vorhergehenden Ansprüche,
**dadurch gekennzeichnet,**
**dass** der Sensor (8) bzw. dessen Ansteuerelektronik (10a) und die Röntgenstrahlungsquelle von der gleichen Stromversorgungsquelle versorgt sind.

11. Zahnärztliches Röntgensystem nach einem der vorhergehenden Ansprüche,
**dadurch gekennzeichnet,**
**dass** der Sensor (8) synchron mit einer Aktivierung der Röntgenstrahlungsquelle ausgelesen wird.

12. Zahnärztliches Röntgensystem nach Anspruch 11,
**dadurch gekennzeichnet,**
**dass** in der Zentraleinheit (4) des Röntgensystems eine Signal-Einheit (10b) für den Sensor (8) angeordnet ist, welche bei einer Aktivierung der Röntgenstrahlungsquelle ein Startsignal an die Ansteuerelektronik (10a) übermittelt.

13. Zahnärztliches Röntgensystem Anspruch 7 oder 8 und Anspruch 12,
**dadurch gekennzeichnet,**
**dass** die Signal-Einheit (10b) einen USB-Hub beinhaltet.

14. Zahnärztliches Röntgensystem nach einem der vorhergehenden Ansprüche,
**dadurch gekennzeichnet,**
**dass** ferner ein externer PC zur Auswertung der von dem Sensor (8) gelieferten Daten vorgesehen ist.

15. Zahnärztliches Röntgensystem nach Anspruch 14,
**dadurch gekennzeichnet,**
**dass** die Übermittlung der Sensordaten an den PC drahtlos erfolgt.

16. Zahnärztliches Röntgensystem nach Anspruch 14,
**dadurch gekennzeichnet,**
**dass** die Übermittlung der Sensordaten an den PC über ein Datennetzwerk erfolgt.

17. Zahnärztliches Röntgensystem nach Anspruch 14,
**dadurch gekennzeichnet,**
**dass** die Übermittlung der Sensordaten an den PC mittels Speichermedien erfolgt.

18. Zahnärztliches Röntgensystem nach einem der vorhergehenden Ansprüche,
**dadurch gekennzeichnet,**
**dass** der Sensor (8) ein strahlungsempfindliches Halbleiterelement (21) aufweist, welches in einem flachen Gehäuse (20) gelagert ist.

19. Zahnärztliches Röntgensystem nach Anspruch 18,
**dadurch gekennzeichnet,**
**dass** es sich bei dem strahlungsempfindlichen Halbleiterelement (21) um einen CCD-oder einen CMOS-Chip handelt.

20. Zahnärztliches Röntgensystem nach einem der vorhergehenden Ansprüche,
**dadurch gekennzeichnet,**
**dass** das System mindestens zwei Sensoren (8-1, 8-2) aufweist, die gleichzeitig betreibbar sind.

21. Zahnärztliches Röntgensystem nach Anspruch 20,
**dadurch gekennzeichnet,**
**dass** eine in der Zentraleinheit (4) angeordnete Einheit (10b) anhand der von den Sensoren (8-1, 8-2) erfassten Signale automatisch erkennt, welcher Sensor (8-1., 8-2) im Bereich der Röntgenstrahlung angeordnet ist.

22. Zahnärztliches Röntgensystem nach Anspruch 20 oder 21,
**dadurch gekennzeichnet,**
**dass** sich die Sensoren (8-1, 8-2) hinsichtlich ihrer Größe unterscheiden.

## Claims

1. Dental X-ray system having a radiation source, arranged in an X-ray head (2), for producing X-radiation, and an electronic sensor (8) for detecting the X-radiation, wherein the sensor (8) is releasably connectable with a central unit (4) of the X-ray system via a plug connection (11a, 23),
**characterised in that**,
the control electronics (10a) required for the operation of the sensor (8) is integrated into a plug (11a) associated with the sensor (8) and connected therewith via an external connector cable (11).

2. Dental X-ray system according to claim 1,
**characterised in that**,
a connector socket (23) for the plug (11a) is arranged in the X-ray head (2) or in a linkage assembly (3) of a mounting carrying the X-ray head (2), in immediate vicinity of the X-ray head (2).

3. Dental X-ray system according to claim 2,
**characterised in that**,
one or a plurality of connection lines (9) extend from the connector socket (23) to the central unit (4) of the X-ray system inside (3) the linkage assembly.

4. Dental X-ray system according to claim 2 or 3,
**characterised in that**,
a repository (13) for the sensor (8) is provided on the X-ray head (2) or the linkage assembly (3).

5. Dental X-ray system according to claim 4,
**characterised in that**,
the repository (13) is removable.

6. Dental X-ray system according to claim 4 or 5,
**characterised in that**,
the repository (13) is of sterilizable material.

7. Dental X-ray system according to any of claims 1 to 6,
**characterised in that**,
the plug connection (11a, 23) contains a USB connection.

8. Dental X-ray system according to claim 7,
**characterised in that**,
the control electronics (10a) integrated into the plug (23) is configured as a USB device.

9. Dental X-ray system according to any of claims 1 to 6,
**characterised in that**,
the plug connection (11a, 23) contains a firewire connection or a connection in accordance with another PC standard.

10. Dental X-ray system in accordance with any preceding claim,
**characterised in that**,
the sensor (8) or its control electronics (10a) and the X-radiation source are supplied from the same power supply source.

11. Dental X-ray system in accordance with any preceding claim,
**characterised in that**,
the sensor (8) is read out synchronously with an activation of the X-radiation source.

12. Dental X-ray system according to claim 11,
**characterised in that**,
there is arranged in the central unit (4) of the X-ray system a signal unit (10b) for the sensor (8) which transmits a start signal to the control electronics (10a) upon an activation of the X-radiation source.

13. Dental X-ray system claim 7 or 8 and claim 12,
**characterised in that**,
the signal unit (10 b) contains a USB hub.

14. Dental X-ray system in accordance with any preceding claim,
**characterised in that**,
further an external PC is provided for the evaluation of the data provided by the sensor (8).

15. Dental X-ray system according to claim 14,
**characterised in that**,
the transmission of the sensor data to the PC is effected wirelessly.

16. Dental X-ray system according to claim 14,
**characterised in that**,
the transmission of the sensor data to the PC is effected via a data network.

17. Dental X-ray system according to claim 14,
**characterised in that**,
the transmission of the sensor data to the PC is effected by means of storage media.

18. Dental X-ray system in accordance with any preceding claim,
**characterised in that**,
the sensor (8) has a radiation sensitive semiconductor element (21) which is mounted in a flat housing (20).

19. Dental X-ray system according to claim 18,
**characterised in that**,
the radiation sensitive semiconductor element (21) is a CCD chip or a CMOS chip.

20. Dental X-ray system in accordance with any preceding claim,
**characterised in that**,
the system has at least two sensors (8-1, 8-2), which are simultaneously operable.

21. Dental X-ray system according to claim 20,
**characterised in that**,
a unit (10b) arranged in the central unit (4) automatically recognizes, on the basis of the signals detected by the sensors (8-1, 8-2), which sensor (8-1, 8-2) is arranged in the region of the X-radiation.

22. Dental X-ray system according to claim 20 or 21,
**characterised in that**,
the sensors (8-1, 8-2) are different with regard to their size.

## Revendications

1. Système de radiographie dentaire avec une source de rayonnement disposée dans une tête radiographique (2) pour la production d'un rayonnement radiographique, ainsi qu'un capteur électronique (8) pour la détection du rayonnement radiographique, le capteur (8) pouvant être relié de manière amovible au moyen d'une borne enfichable (11a, 23) à une unité centrale (4) du système de radiographie,
**caractérisé en ce que**
l'électronique de commande (10a) nécessaire pour le fonctionnement du capteur (8) est intégrée dans une prise (11a) affectée au capteur (8) et reliée à celui-ci au moyen d'un câble de connexion (11).

2. Système de radiographie dentaire selon la revendication 1,
**caractérisé en ce que**
un connecteur (23) pour la prise (11 a) est disposé dans la tête radiographique (2) ou dans une rampe (3) supportant la tête radiographique (2) d'un support à proximité immédiate de la tête radiographique (2).

3. Système de radiographie dentaire selon la revendication 2,
**caractérisé en ce que**
une ou plusieurs lignes de connexion (9) s'étendent du connecteur (23) à l'unité centrale (4) du système de radiographie à l'intérieur (3) de la rampe.

4. Système de radiographie dentaire selon la revendication 2 ou 3,
**caractérisé en ce que**
une tablette (13) est prévue pour le capteur (8) sur la tête radiographique (2) ou la rampe (3).

5. Système de radiographie dentaire selon la revendication 4,
**caractérisé en ce que**
la tablette (13) est amovible.

6. Système de radiographie dentaire selon la revendication 4 ou 5,
**caractérisé en ce que**
la tablette (13) est en matériau stérilisable.

7. Système de radiographie dentaire selon l'une quelconque des revendications 1 à 6,
**caractérisé en ce que**
la borne enfichable (11a, 23) comporte un port USB.

8. Système de radiographie dentaire selon la revendication 7,
**caractérisé en ce que**
l'électronique de commande (10a) intégrée dans le connecteur (23) est réalisée comme un dispositif USB.

9. Système de radiographie dentaire selon l'une quelconque des revendications 1 à 6,
**caractérisé en ce que**
la borne enfichable (11a, 23) comprend un port FireWire ou un port selon une autre norme PC.

10. Système de radiographie dentaire selon l'une quelconque des revendications précédentes,
**caractérisé en ce que**
le capteur (8) et/ou son électronique de commande (10a) et la source de rayonnement radiographique sont alimentés par la même source d'alimentation électrique.

11. Système de radiographie dentaire selon l'une quelconque des revendications précédentes,
**caractérisé en ce que**
le capteur (8) est lu de manière synchrone avec une activation de la source de rayonnement radiographique.

12. Système de radiographie dentaire selon la revendication 11,
**caractérisé en ce que**
dans l'unité centrale (4) du système de radiographie est disposée une unité de signal (10b) pour le capteur (8), laquelle transmet un signal de départ à l'électronique de commande (10a) lorsque la source de rayonnement radiographique est activée.

13. Système de radiographie dentaire selon la revendication 7 ou 8 et la revendication 12,
**caractérisé en ce que**
l'unité de signal (10b) comprend un concentrateur USB.

14. Système de radiographie dentaire selon l'une quelconque des revendications précédentes,
**caractérisé en ce que**
à distance, un PC externe est prévu pour l'exploitation des données fournies par le capteur (8).

15. Système de radiographie dentaire selon la revendication 14,
**caractérisé en ce que**
la transmission des données du capteur au PC s'effectue sans fil.

16. Système de radiographie dentaire selon la revendication 14,
**caractérisé en ce que**
la transmission des données du capteur au PC s'effectue au moyen d'un réseau de données.

17. Système de radiographie dentaire selon la revendication 14,
**caractérisé en ce que**
la transmission des données du capteur au PC s'effectue au moyen de supports de stockage.

18. Système de radiographie dentaire selon l'une quelconque des revendications précédentes,
**caractérisé en ce que**
le capteur (8) comprend un élément semi-conducteur (21) sensible au rayonnement, lequel est logé dans un boîtier plat (20).

19. Système de radiographie dentaire selon la revendication 18,
**caractérisé en ce que**
l'élément semi-conducteur sensible au rayonnement (21) est une puce CCD ou CMOS.

20. Système de radiographie dentaire selon l'une quelconque des revendications précédentes,
**caractérisé en ce que**
le système comprend au moins deux capteurs (8-1, 8-2) qui peuvent fonctionner simultanément.

21. Système de radiographie dentaire selon la revendication 20,
**caractérisé en ce que**
une unité (10b) disposée dans l'unité centrale (4) est capable de déterminer, en fonction des signaux détectés par les capteurs (8-1, 8-2), quel capteur (8-1, 8-2) se trouve dans la zone de rayonnement radiographique.

22. Système de radiographie dentaire selon la revendication 20 ou 21,
**caractérisé en ce que**
les capteurs (8-1, 8-2) se différencient par leur taille.
